Europäisches Patentamt

European Patent Office

Office européen des brevets

(19)

(11) Veröffentlichungsnummer: **0 101 913**

**A2**

(12) **EUROPÄISCHE PATENTANMELDUNG**

(21) Anmeldenummer: 83107242.6

(22) Anmeldetag: 23.07.83

(51) Int. Cl.³: **B 01 J 23/76**
**B 01 J 35/10, C 07 C 15/46**
**C 07 C 5/333**

(30) Priorität: 03.08.82 DE 3228877
03.08.82 DE 3228879
03.08.82 DE 3228878

(43) Veröffentlichungstag der Anmeldung:
07.03.84 Patentblatt 84/10

(84) Benannte Vertragsstaaten:
BE DE FR GB IT NL SE

(71) Anmelder: BASF Aktiengesellschaft
Carl-Bosch-Strasse 38
D-6700 Ludwigshafen(DE)

(72) Erfinder: Biffar, Werner, Dr.
Lucas-Cranach-Strasse 4
D-6710 Frankenthal(DE)

(72) Erfinder: Mross, Wolf Dieter, Dr.
Anselm-Feuerbach-Strasse 21
D-6710 Frankenthal(DE)

(72) Erfinder: Biedenkapp, Dieter, Dr.
Froebelstrasse 26
D-6700 Ludwigshafen(DE)

(72) Erfinder: Schweter, Walter, Dr.
Am Weidenschlag 110
D-6700 Ludwigshafen(DE)

(54) Dehydrierungskatalysator.

(57) Dehydrierungskatalysator aus 50 bis 95 Gew.% einer Einsenverbindung und

5 bis 50 Gew.% mindestens eines Promotors und/oder mindestens eines Stabilisators.

Zur Herstellung des Katalysators werden als Eisenverbindung Goethit, Lepidokrokit, Hämatit, Maghämit, Magnetit oder Mischungen der genannten Stoffe einer spezifischen Oberfläche von 1 bis 150 $m^2/g$, gemessen nach BET, verwendet und mit der(n) weiteren Komponente(n) gemischt oder geknetet und anschließend geformt.

Der Katalysator wird zur Dehydrierung von Ethylbenzol zu Styrol verwendet.

EP 0 101 913 A2

Croydon Printing Company Ltd

BASF Aktiengesellschaft                    O.Z.0050/36072
                                                36073/36074

Dehydrierungskatalysator

Die Erfindung betrifft einen Dehydrierungskatalysator,
enthaltend

a) 50 bis 95 Gew.%, bezogen auf a + b, mindestens einer
   Eisenverbindung, berechnet als $Fe_2O_3$,

und

b) 5 bis 50 Gew.%, bezogen auf a + b, mindestens eines
   Promotors und/oder mindestens eines Stabilisators,
   berechnet als stabilstes Oxid.

Olefine, insbesondere Butadiene und Vinylbenzole sind wichtige Ausgangsprodukte für die Herstellung von Kunststoffen, synthetischen Harzen und Kautschukarten.

Im allgemeinen werden sie bei den technisch ausgeübten
Verfahren durch Überleiten von teilgesättigten Kohlenwasserstoffen z.B. Ethylbenzol oder Buten zusammen mit
Wasserdampf über einen Festbettkatalysator bei Temperaturen zwischen 450 und 700°C dehydriert. Das Verfahren
kann isotherm oder adiabatisch ausgeführt werden (vgl. 1).

Zum Stand der Technik nennen wir:

(1) Kunststoff-Handbuch, Band V, Polystyrol, Carl Hanser-
    -Verlag, Seiten 39 bis 47 (1969),
(2) US-PS 4 144 197,
(3) DE-OS 25 44 185 und
(4) deutsche Patentanmeldung P 31 32 014.7

Vo/ro

Als Katalysatoren werden Eisenoxide, promotiert mit oxid-bildenden Alkalisalzen und weiteren selektivitäts-steigernden und strukturstabilisierenden Metallverbindungen verwendet. Zur Herstellung des Katalysators können Eisenoxide, die durch thermische Zersetzung von Eisennitrat, Eisenoxalat oder Eisensulfat gewonnen werden ebenso eingesetzt werden wie technisch in großem Maßstab produzierte Eisenoxidpigmente, z.B. Eisenrot (Hämatit-alpha-$Fe_2O_3$) und Eisengelb (Geothit-alpha-FeOOH).

Das gemahlene Eisenoxid - 50 bis 95 Gew.% der Kontaktmasse - wird mit den pulverförmigen oder in Wasser gelösten Promotoren unter weiterem Zusatz von Wasser vermengt und zu einer plastischen Masse geknetet, zu Strängen extrudiert, getrocknet und bei Temperaturen zwischen 500 und 1000°C (in aller Regel im Bereich zwischen 850 und 950°C) 0,5 bis 3 Stunden calciniert [vgl. (2), (3) und (4)]. Dabei wird eine Kontaktoberfläche zwischen 3 und 4 $m^2$/g angestrebt.

In den so hergestellten Katalysatoren liegt das Eisenoxid nach Röntgenbeugungsaufnahmen als Hämatit (alpha-$Fe_2O_3$) durchsetzt mit Kaliumferraten und anderen Promotorsalzen vor.

Zu Beginn der Reaktion wird der Katalysator durch das eingeleitete Ethylbenzol reduziert. Es bildet sich der in Wasserdampf/Wasserstoff-Atmosphäre thermodynamisch stabile Magnetit, $Fe_3O_4$.

Bislang ging man davon aus, daß die bei der Reduktion zwangsläufig eintretende Umwandlung des Sauerstoffgerüstes im Eisenoxid von einer hexagonalen (alpha-$Fe_2O_3$) in eine kubisch dichteste Kugelpackung ($Fe_3O_4$) eine notwendige Voraussetzung für die Aktivierung des Katalysators sei, da

BASF Aktiengesellschaft        - 3 -        O.Z. 0050/36072/
                                                  36073/36074

dieser Prozeß einen erheblichen Anstieg der Störstellen und Fehlordnungen im Eisenoxidgitter zur Folge haben sollte (vgl. E. Lichtner und A. Weiss, Z. anorg. allg. Chem. $\underline{359}$, 214 bis 219 (1968)). Demnach war die Verwendung von Magnetit oder Maghämit ($\gamma$ Fe$_2$O$_3$) als Eisenoxidkomponente nur dann möglich, wenn man im Falle des Magnetits durch oxidierendes Calcinieren bei Temperaturen über 700°C die Umwandlung in Hämatit ($\alpha$ Fe$_2$O$_3$) durchführte. Bezüglich der Verwendung anderer Oxide werden der Temperaturbehandlung (Calcinierung) werden nach Angaben in der Literatur eine Reihe wichtiger Aufgaben im Zuge der Katalysatorherstellung zugesprochen:

1. Bei der Calcinierung wandeln sich oxidbildende Ausgangsstoffe (z.B. Oxidhydrate, Carbonate, Nitrate etc.) in die entsprechenden Oxide um.

2. Chemische Feststoffbrücken werden gebildet, die die Erhöhung der mechanischen Festigkeit der Katalysatorpellets bedingen.

3. Die Katalysatoroberfläche wird auf einen BET-Wert unter 8 m$^2$/g gesenkt. Dies wird als eine Voraussetzung für die hohen Selektivitätseigenschaften des Katalysators angesehen.

Überraschend haben wir nunmehr gefunden, daß man durchaus von Magnetit, Maghämit, Hämit und/oder Goethit bei der Herstellung eines aktiven Dehydrierkatalysators ausgehen kann, ohne den Umweg über eine Calcinierung - im Falle des Magnetits mit Luftoxidation - gehen zu müssen.

Die Erfindung betrifft daher einen Dehydrierungskatalysator, enthaltend

BASF Aktiengesellschaft — 4 — O.Z. 0050/36072/
36073/36074

a) 50 bis 95 Gew.%, vorzugsweise von 60 bis 91 Gew.%,
bezogen auf a + b, mindestens einer Eisenverbindung,
berechnet als $Fe_2O_3$,

und

b) 5 bis 50 Gew.%, vorzugsweise von 10 bis 40 Gew.%, bezogen auf a + b, mindestens eines Promotors und/oder
mindestens eines Stabilisators, berechnet als stabilstes Oxid,
gegebenenfalls enthaltend
c) übliche Zusätze.

<u>dadurch gekennzeichnet</u>, daß man zur Herstellung des Katalysators als Komponente a) Goethit, Lepidokrokit, Hämatit,
Maghämit, Magnetit, oder Mischungen der genannten Stoffe,
einer spezifischen Oberfläche von 1 bis 150 $m^2/g$, vorzugsweise von 2 bis 40 $m^2/g$, gemessen nach BET, verwendet und
die Komponente a) mit der(n) weiteren Komponente(n) mischt
und anschließend formt.

Vorteile der Erfindung:
- Unabhängig von der Wahl des eingesetzten Eisenoxids
  (Goethit, Lepidokrokit, Hämatit, Maghämit, Magnetit
  etc.) gelingt eine wesentliche Vereinfachung der Katalysatorherstellung. So genügt selbst einfaches
  trockenes Mischen der Komponenten und anschließendes
  Tablettieren.
- Die eingangs genannten Produktionsschritte des Knetens
  und Calcinierens können entfallen, was zu einer
  Energiekosteneinsparung führt.
- Die Auswahl verschiedener Eisenoxidpigmente, die preisgünstig auf dem Markt angeboten werden, steht offen.
- Die Einflüsse der zugesetzten Promotoren und/oder Stabilitsatoren bleiben unverändert wirksam; ein Quali- tätsverlust kann daher ausgeschlossen werden. Bezüglich der Art der Promotoren, der Stabilisatoren und
Zusätze und deren Definition sei auf (1), (2) und insbesondere (4) verwiesen.

Speziell für die Verwendung von Eisenoxiden mit kubischem
Oxidgitter (z.B. Magnetit, Maghämit) gilt außerdem:

- Nach der Formgebung entfällt eine Phasenumwandlung des
  Oxidgitter zu Beginn der Dehydrierreaktion. Dies führt
  zu einer erhöhten mechanischen Festigkeit der Pellets
  über die gesamte und mithin längere Laufzeit des Katalysators.

Zur Herstellung des erfindungsgemäßen Katalysators soll
als Komponente a) Magnetit, Maghämit, Goethit ($\alpha$-FeOOH),
Lepidokrokit ($\alpha$-FeOOH) oder Hämatit ($\gamma$-Fe$_2$O$_3$) oder
Mischungen aus diesen verwendet werden, der BET-Oberfläche
1 bis 150 m$^2$/g beträgt. Vorzuziehen ist ein Oberflächenbereich von 4 bis 40 m$^2$/g und insbesondere ein solcher
zwischen 10 und 25 m$^2$/g. (Die BET-Oberfläche wird gemessen
nach Brunauer, Emett, Teller, J. Amer. Chem. Soc. 60
(1938), Seite 309.) Die einfachste Herstellungsweise besteht im trockenen Mischen der pulverförmigen
Komponente a) mit den ebenfalls klein gemahlenen Promotoren und anschließende Tablettierung nach Zugabe eines
Tablettierhilfsmittels (z.B. Graphit) zu mechanisch stabilen Formkörpern. Die Promotoren können in oxidischer Form
oder als oxidbildende Metallsalze insbesondere als Carbonate und Hydroxide eingesetzt werden.

Neben rein trockenen Mischungen können auch Knetmassen aus
der Komponente a), z.B. dem Magnetit- oder $\gamma$-Eisenoxid-
pigment, und den Promotoren unter Zusatz von Wasser hergestellt werden, die, zu Strängen extrudiert, bei Tempera-

0101913

turen bis zu 200°C gegebenenfalls unter Vakuum getrocknet
werden. Wäßrige Suspensionen der Kontaktmischung können
nach Sprühtrocknung tablettiert werden.

Als Tablettenformen eignen sich massive Zylinder mit 3 bis
10 mm Durchmesser und einer Länge von 3 bis 10 mm. Aufgrund kinetischer und betriebstechnischer Bedingungen sind
Ringtabletten mit vergleichbaren Außendurchmessern der
Tablettenform vorzuziehen. Gleiches gilt für Extrudate, wo
ebenfalls Hohlsträngen der Vorzug zu geben ist.

Beispiele zur Herstellung erfindungsgemäßer Katalysatoren:

Die Herstellung erfolgt nach zwei Methoden:

A: Eisenoxidpulver wird nach Zusatz von Promotoren mit
Wasser angeteigt und anschließend entweder nach Formgebung (Extrudieren) getrocknet oder nach Trocknung
tablettiert.

B: Eisenoxidpulver wird mit fein gemahlenen Metallsalzen
als Promotoren trocken gemischt, 2 % Graphit zugesetzt
und tablettiert.

In den Tabellen 1, 2, 3 und 4 sind für eine Reihe erfindungsgemäßer Katalysatoren Herstellungsart (A oder B)
Eisenoxid und Zusammensetzung, sowie deren Selektivität
angegeben. In der Tabelle 5 sind die Bedingungen für die
Herstellung der Katalysatoren 1 und 2 von Tabelle 3
(Methode A), angegeben. In Tabelle 6 sind die Bedingungen
für die Herstellung der Katalysatoren 1 bis 5 von
Tabelle 4 (Methode A) aufgeführt.

0101913

Beispiel 1 bis 41

Testvorschrift für die erfindungsgemäßen Katalysatoren zur beispielhaften Dehydrierung von Ethylbenzol zu Styrol.

Die in den Tabellen 2, 3 und 4 beschriebenen Katalysatoren, hergestellt nach Methode A, in Form von Tabletten und die Katalysatoren, hergestellt nach Methode B wurden auf eine Körnung von 0,5 bis 1 mm zerkleinert. Von diesen Kontaktmassen wurden jeweils 20 ml in einen Reaktor mit 6 mm Innendurchmesser eingefüllt. Pro Stunde wurden 10 ml Wasser und 10 ml Ethylbenzol in den Reaktor dosiert. Die Temperatur des zur Reaktorheizung verwendeten Salzbades wurde jeweils so eingestellt, daß die organische Phase des Reaktoraustrages 50 % Styrol enthält. Nach 6 Tagen wurde eine Gesamtanalyse der organischen Phase und des Abgases durchgeführt und die Selektivität des Katalysators errechnet.

Tabelle 1

| Kata-lysa-sator | Eisen-oxid | Herstel-lungs-methode | Herstellungsbedingungen bei Methode A | | | |
|---|---|---|---|---|---|---|
| | | | Wasser-zugabe [ml] | Zeit [h] | Trocknung Temp. [$^{\circ}$C] | Druck [mb] |
| 1 | Magnetit | A | 80 | 2/22 | 130/100 | 1000 |
| 2 | " | A | 127 | 20 | 90 | 1000 |
| 3 | " | A | 184 | 19 | 40 | 200 |
| 4 | " | A | 30 | 16 | 80 | 200 |
| 5 | " | A | 46 | 16 | 80 | 200 |
| 6 | " | A | 44 | 16 | 80 | 200 |
| 7 | " | A | 135 | 20 | 90 | 1000 |
| 8 | " | A | 180 | 20 | 90 | 1000 |
| 9 | " | A | 97 | 2/22 | 130/100 | 1000 |
| 10 | " | A | 40 | 24 | 130 | 1000 |

0101913

Tabelle 1 (Fortsetzung)

| Kata-lysa-sator | Eisen-oxid | Herstel-lungs-methode | Herstellungsbedingungen bei Methode A | | | |
| | | | Wasser-zugabe [ml] | Zeit [h] | Trocknung Temp. [$^{\circ}$C] | Druck [mb] |
|---|---|---|---|---|---|---|
| 11 | Magnetit | A | 97 | 2/22 | 130/100 | 1000 |
| 12 | " | A | 108 | 2/22 | 130/100 | 1000 |
| 13 | " | A | 180 | 19 | 40 | 200 |
| 14 | " | A | 164 | 19 | 40 | 200 |
| 15 | " | B | - | - | - | - |
| 16 | " | B | - | - | - | - |
| 17 | " | B | - | - | - | - |
| 18 | " | B | - | - | - | - |
| 19 | " | B | - | - | - | - |
| 20 | " | B | - | - | - | - |
| 21 | " | B | - | - | - | - |

## Tabelle 2

| Kataly-sator | Komponenten des Katalysators in Teilen | | | | Testung a)Reaktions-temperatur [°C] | b)Selekti-vität [%] |
|---|---|---|---|---|---|---|
| | Magnetit | Promotor bzw. Stabilisator | | | | |
| 1 | 100 (90,5) M-20[c] | 14,7 (9,5) KOH[d] | | | 580 | 92,37 |
| 2 | 100 (89,2) M-20 | 17,0 (10,8) KOH | | | 580 | 91,75 |
| 3 | 100 (80,4) M-22 | 37,0 (19,6) $K_2CO_3$ | | | 576 | 92,23 |
| 4 | 100 (89,2) M-4 | 17,0 (10,8) KOH | | | 597 | 90,90 |
| 5 | 100 (89,2) M-10 | 17,0 (10,8) KOH | | | 578 | 89,11 |
| 6 | 100 (89,2) M-15 | 17,0 (10,8) KOH | | | 572 | 91,04 |
| 7 | 100 (89,2) M-22 | 17,0 (10,8) KOH | | | 581 | 92,44 |
| 8 | 100 (89,2) M-39 | 17,0 (10,8) KOH | | | 599 | 95,58 |
| 9 | 100 (89,1) M-20 | 9,7 (7,0) KOH | 5,9 (3,9) NaOH[d] | | 578 | 90,10 |
| 10 | 100 (87,5) M-20 | 17,2 (10,8) KOH | 1,2 (0,6) $Li_2CO_3$ | 1,5 (1,07) AlOOH | 573 | 90,28 |
| 11 | 100 (89,8) M-20 | 14,7 (9,4) KOH | 0,9 (0,8) $V_2O_5$ | | 608 | 94,97 |
| 12 | 100 (88,4) M-20 | 14,7 (9,3) KOH | 1,5 (1,3) $V_2O_5$ | 1,2 (1,0) $WO_3$ | 600 | 95,55 |
| 13 | 100 (86,9) M-22 | 17,0 (10,6) KOH | 1,7 (1,4) $V_2O_5$ | 1,3 (1,1) $WO_3$ | 613 | 95,65 |
| 14 | 100 (86,9) M-38 | 17,0 (10,6) KOH | 1,7 (1,4) $V_2O_5$ | 1,3 (1,1) $WO_3$ | 606 | 92,25 |
| 15 | 100 (90,5) M-20 | 14,7 (9,5) KOH | | | 607 | 93,39 |
| 16 | 100 (89,2) M-20 | 18,5 (10,8) $K_2CO_3$ | | | 600 | 93,12 |
| 17 | 100 (88,8) M-14 | 19,2 (11,2) $K_2CO_3$ | | | 600 | 93,50 |
| 18 | 100 (85,6) M-11 | 19,2 (10,8) $K_2CO_3$ | 1,1 (0,9) $V_2O_5$ | 3,3 (2,7) $WO_3$ | 627 | 94,31 |
| 19 | 100 (85,6) M-10 | 19,2 (10,8) $K_2CO_3$ | 1,1 (0,9) $V_2O_5$ | 3,3 (2,7) $WO_3$ | 620 | 95,86 |
| 20 | 100 (85,6) M-14 | 19,2 (10,8) $K_2CO_3$ | 1,1 (0,9) $V_2O_5$ | 3,3 (2,7) $WO_3$ | 614 | 95,88 |
| 21 | 100 (85,6) M-12 | 19,2 (10,8) $K_2CO_3$ | 1,1 (0,9) $V_2O_5$ | 3,3 (2,7) $WO_3$ | 616 | 95,73 |

alle Werte in Klammern sind Gew.%, bezogen auf a+b, unter Annahme des stabilsten Oxids, z.B. $Fe_2O_3$, $K_2O$, $Na_2O$, $Li_2O$, $Al_2O_3$, $V_2O_5$, $WO_3$

a) Reaktionstemperatur bei 50 % Styrol im organischen Teil des Ofenöls
b) Styrolselektivität, bezogen auf eingesetztes Ethylbenzol
c) M-x: M = Magnetit; x = Oberflächenwert des Magnetits (angegeben in $m^2$/g)

d) als Pulver eingesetzt

## Tabelle 3

|  |  | Katalysator | Testergebnisse | |
|---|---|---|---|---|
| Nr. | Herst.-Methode | Katalysatorzusammensetzung [g] bezogen auf a + b unter Annahme der Oxide $Fe_2O_3$, $K_2O$, $Li_2O$, $V_2O_5$, $WO_3$, $Al_2O_3$, $Cr_2O_3$) | Reaktions-temperatur [°C](a) | Styrol-selekti-vität [%](b) |
| 1 | A | 100 (87,0)[x] $\alpha$-FeOOH 15,96 (10,5) $K_2CO_3$ 1,46 (1,4) $V_2O_5$ 1,15 (1,1) $WO_3$ | 601 | 95,93 |
| 2 | A | 100 (87,0) $\alpha$-FeOOH 15,96 (10,5) $K_2CO_3$ 1,46 (1,4) $V_2O_5$ 1,15 (1,1) $WO_3$ | 593 | 96,18 |
| 3 | B | 100 (87,0) $\alpha$-FeOOH 15,96 (10,5) $K_2CO_3$ 1,46 (1,4) $V_2O_5$ 1,15 (1,1) $WO_3$ | 594 | 96,26 |
| 4 | B | 100 (85,9) $\alpha$-FeOOH 15,96 (10,4) $K_2CO_3$ 1,46 (1,4) $V_2O_5$ 1,15 (1,1) $WO_3$ 1,5 (1,2) AlOOH | 606 | 95,53 |
| 5 | B | 100 (84,9) $\alpha$-FeOOH 15,96 (10,3) $K_2CO_3$ 1,46 (1,4) $V_2O_5$ 1,15 (1,1) $WO_3$ 2,47 (2,3) $Cr_2O_3$ | 603 | 95,49 |
| 6 | B | 100 (86,6) $\alpha$-FeOOH 15,96 (10,5) $K_2CO_3$ 1,46 (1,4) $V_2O_5$ 1,15 (1,1) $WO_3$ 1,15 (0,4) $Li_2CO_3$ | 595 | 96,15 |
| 7 | B | 100 (86,1) $\alpha$-FeOOH 15,96 (10,4) $K_2CO_3$ 0,90 (0,9) $V_2O_5$ 2,72 (2,6) $WO_3$ | 610 | 95,97 |
| 8 | B | 100 (85,7) $\alpha$-$Fe_2O_3$ 18,4 (10,7) $K_2CO_3$ 1,04 (0,9) $V_2O_5$ 3,14 (2,7) $WO_3$ | 611 | 95,67 |

a) Reaktionstemperatur bei 50 % Styrol im organischen Teil des Ofenöls;

b) Styrolselektivität, bezogen auf eingesetztes Ethylbenzol

in ()[x] Werte in Gew.%, bezogen auf a + b unter Annahme der Oxide

$Fe_2O_3$, $K_2O$, $Li_2O$, $V_2O_5$, $WO_3$, $Al_2O_3$, $Cr_2O_3$)

O.Z. 0050/36074
36073/36074

0101913

O.Z. 0050/36073
36073/36074

Tabelle 4

| Nr. | Her-stellungs-methode | Katalysatorzusammensetzung [g] (Gew.% bezogen auf a + b unter Annahme der Oxide $Fe_2O_3$, $K_2O$, $CrO_3$, $V_2O_5$, $WO_3$) | Reaktions-temperatur [°C] [a] | Styrol-selektivität [%] [b] |
|---|---|---|---|---|
| 1 | A | 100 (85,6) α-20[c] 18,6 (10,8) $K_2CO_3$ 1,1 (0,9) $V_2O_5$ 3,1 (2,7) $WO_3$ | 595 | 95,33 |
| 2 | A | 100 (84,3) α-20 17,1 (12,1) KOH[d] 1,1 (0,9) $V_2O_5$ 3,1 (2,7) $WO_3$ | 598 | 94,91 |
| 3 | A | 100 (85,6) α-20 22,6 (10,8) $K(HCO_2)$ 1,1 (0,9) $V_2O_5$ 3,1 (2,7) $WO_3$ | 597 | 95,84 |
| 4 | A | 100 (84,3) α-16 18,6 (10,7) $K_2CO_3$ 1,8 (1,5) $CrO_3$ 1,1 (0,9 $V_2O_5$ 3,1 (2,6) | 596 | 96,05 |
| 5 | A | 100 (84,3) α-16 18,6 (10,7) $K_2CO_3$ 1,8 (1,5) $CrO_3$ 1,1 (0,9) $V_2O_5$ 3,1 (2,6) $WO_3$ | 600 | 95,34 |
| 6 | B | 100 (88,8) α-20 18,6 (11,2) $K_2CO_3$ | 592 | 94,51 |
| 7 | B | 100 (88,8) α-16 18,6 (11,2) $K_2CO_3$ | 567 | 88,94 |
| 8 | B | 100 (85,6) α-20 22,6 (10,8) $K(HCO_2)$ 1,1 (0,9) $V_2O_5$ 3,1 (2,7) $WO_3$ | 607 | 95,21 |
| 9 | B | 100 (85,6) α-16 18,6 (10,8) $K_2CO_3$ 1,1 (0,9) $V_2O_5$ 3,1 (2,7) $WO_3$ | 595 | 96,77 |
| 10 | B | 100 (85,6) α-16 18,6 (10,8) $K_2CO_3$ 1,1 (0,9) $V_2O_5$ 3,1 (2,7) $WO_3$ | 595 | 96,50 |
| 11 | B | 100 (84,3) α-16 18,6 (10,7) $K_2CO_3$ 1,8 (1,5) $CrO_3$ 1,1 (0,9) $V_2O_5$ 3,1 (2,6) $WO_3$ | 594 | 95,34 |

a) Reaktionstemperatur bei 50 % Styrol im organischen Teil des Ofenöls;
b) Styrolselektivität, bezogen auf eingesetztes Ethylbenzol;
c) α-X: α = $\gamma$-$Fe_2O_3$; X = Oberflächenwert des $\gamma$-$Fe_2O_3$ (angegeben in $m^2/g$);
d) als Pulver eingesetzt.

Tabelle 5

| Katalysator | Eisen-oxid | Wasser-zugabe [ml] | Trocknung Zeit [h] | Trocknung Temperatur [°C] |
|---|---|---|---|---|
| 1 | Goethit | 126 | 24 | 100 |
| 2 | " | 126 | 12 | 300 |

Tabelle 6

| Kataly-sator | Eisen-oxid | Wasser-zugabe [ml] | Trocknung Zeit [h] | Trocknung Temp. [°C] | Bemerkungen |
|---|---|---|---|---|---|
| 1 | Maghämit | 120 | 16 | 100 | – |
| 2 | " | 112 | 16 | 100 | – |
| 3 | " | 90 | 16 | 100 | – |
| 4 | " | 50 | 24 | 100 | – |
| 5 | " | 45 | 24 | 100 | Die Komponenten wurden 3 h geknetet, zu 1 mm-Strängen extrudiert, getrocknet, gemahlen und nach Zusatz von 2 % Graphit tablettiert. |

Beispiele 42 und 43

Diese Beispiele sollen belegen, daß Ringtabletten, verglichen mit Tabletten Vorteile besitzen.

Unter vergleichbaren Reaktionsbedingungen wurden Vollstränge mit dem Durchmesser von 6 mm und 10 mm Länge und Ringtabletten mit 7,5 mm Dicke und dem Durchmesser
7,5 mm (außen) bzw. 3,0 mm (innen) gleicher Kontaktmasse
getestet. Stränge und Tabletten hatten das gleiche Schüttgewicht von 1100 g/l. Dabei wurde eine Selektivitätssteigerung von 0,7 bis 1,2 % und ein Aktivitätsgewinn von
5 bis 10°C beim Übergang vom Strang zur Ringtablette beobachtet.

BASF Aktiengesellschaft — 14 — O.Z. 0050/36072/ 36073/36074

Patentansprüche

1. Dehydrierungskatalysator, enthaltend

a) 50 bis 95 Gew.%, bezogen auf a + b, mindestens einer Eisenverbindung, berechnet als $Fe_2O_3$,

und

b) 5 bis 50 Gew.%, bezogen auf a + b, mindestens eines Promotors oder mindestens eines Stabilisators oder Mischungen von Promotor und Stabilisator, berechnet als stabilstes Oxid,

gegebenenfalls enthaltend

c) übliche Zusätze,

dadurch gekennzeichnet, daß man zur Herstellung des Katalysators als Komponente a) Goethit, Lepidokrokit, Hämatit, Maghämit, Magnetit oder Mischungen der genannten Stoffe einer spezifischen Oberfläche von 1 bis 150 $m^2$/g gemessen nach BET, verwendet und die Komponente a) mit der(n) weiteren Komponente(n) mischt und anschließend formt.

2. Verwendung des Katalysators gemäß Anspruch 1 zur Dehydrierung von Ethylbenzol zu Styrol.